# EUROPEAN PATENT APPLICATION

(11) **EP 0 739 904 A1**
(43) Date of publication of application: **30.10.1996**
(21) Application number: 96201024.5
(22) Date of filing: 29.06.1990
(51) Int. Cl.: C07K 16/46, C07K 16/28, C07K 16/10, C12N 5/00, A61K 39/395, A61K 35/12

(54) **Bispecific reagents for aids therapy**

(30) Priority: 29.06.1989 US 373905
(62) Divisional of application: 90911182.5
(71) Applicant: MEDAREX, INC., Princeton, NJ 08542 (US)
(72) Inventor: Fanger, Michael W., Lebanon, New Hampshire 03766 (US); Guyre, Paul M., Hanover, New Hampshire 03755 (US); Dinces, Nathan B., Canaan, New Hampshire (US)
(74) Representative: Holdcroft, James Gerald, Dr.

(57) **Abstract**

Bispecific molecules which react both with the high-affinity Fcγ receptor of human effector cells and with a virus or virus component are disclosed. Binding of the molecules to the Fc receptors found on effector cells is not blocked by human immunoglobulin G. The molecules are useful for targeting human effector cells (e.g. macrophages) against a viral target (e.g. HIV or BIV-infected cell). For this purpose, bispecific molecules can be constructed containing the binding region derived from an anti-Fcγ receptor antibody and the CD4 molecule or CD4 binding domain of the envelope glycoprotein gp120 of BIV. Alternatively, bispecific antibodies or heteroantibodies can be constructed containing the binding region derived from an anti-Fc receptor antibody and the binding region of HIV-specific antibody such as anti-gp120 antibody. Targeted effector cells can be used to kill virus by cell mediated antibody dependent cytolysis.

## Description

In the absence of an effective vaccine or therapy, the incidence of acquired immune deficiency syndrome (AIDS) in the United States and other countries is likely to increase during the next few years. Preventing infection with the human immunodeficiency virus (HIV) will depend upon education and counselling to prevent transmission among the populations at risk for AIDS.

To date, neither active immunization with the HIV envelope glycoprotein gp120 nor passive immunization with AIDS-immune serum has protected non-human primates from subsequent challenge with AIDS. The prospects for effective immunization against HIV infection are not encouraging at this time.

Recently, the initial events in infection of human T lymphocytes, macrophages, and other cells by HIV have been elucidated. These events involve the attachment of the HIV envelope glycoprotein gp120 to its cellular receptor, CD4. Cells that lack CD4 are not susceptible to HIV infection, but become susceptible after they are transfected with the CD4 gene and express CD4 on their surfaces. This information has led to studies of use of recombinant CD4 (rCD4) which might be used therapeutically to block the CD4-binding sites on HIV, preventing it from binding to CD4 on host cells. However, this would provide only a passive blockage of virus infection, and would not lead to active elimination of the virus.

A therapeutic approach has been developed to eliminate the virus. This involves linkage of CD4 to the Fc region of human IgG. Capon, D.J. et al. Nature, 337, 525 (1989). The Fc region of human IgG is the natural ligand for receptors on monocytic cells. moreover, in the Fc portion of IgG reside immunoglobulin functions such as Fc receptor binding, protein A binding and complement fixation. These properties of the Fc portion of human immunoglobulin are the major mechanisms for elimination of pathogens. Fc activates the complement pathway, resulting in lysis of the pathogen, whereas binding to the Fc cell receptors on effector cells can lead to ingestion of the pathogen by phagocytosis or lysis by killer cells.

Nevertheless, the vast amount and diversity of natural antibodies (i.e. non-HIV specific IgG) found in vivo remains a major obstacle to this kind of in vivo therapy since non-HIV specific IgG would be expected to block binding of the Fc region with Fc receptors. A need exists to develop a therapeutic modality that overcomes these problems.

This invention pertains to bispecific molecules which can bind a pathogen and which can simultaneously target the pathogen and pathogen-infected cells for ingestion and destruction by effector cells such as monocytes, macrophages, and neutrophils. The bispecific molecules of this invention have a first binding specificity for a pathogen (e.g. virus) and a second binding specificity for the high-affinity Fcγ receptor. The binding specificity for the Fcγ receptor is for a site which is distinct from the ligand binding site for the Fc region of IgG. The bispecific molecules are capable of binding to IgG-occupied receptor of effector cells in the presence of normal serum IgG.

For example, if the target pathogen is a virus such as HIV, the targeted viral component can be the envelope glycoprotein gp120 of HIV. The binding specificity for gp120 can be provided in several ways. It can be provided by the CD4 molecule of T cells or just the CD4 binding domain thereof. Alternatively, the gp120 specificity can be provided by a gp120-specific antibody. The binding specificity for the high affinity Fcγ receptor is provided by an antibody which binds to an epitope of the Fc receptor, the binding of which is not blocked by human IgG.

The bispecific molecules of this invention can be administered alone or they can be pre-attached to effector cells for administration to the patient. They can also be used in conjunction with other molecules. For example, molecules of this invention can be used with cytokines such as interferon-γ which can activate or enhance their therapeutic potential. The effector cells can be obtained from the patient or from other sources so long as the cells are compatible with the patient's immune system. The binding of bispecific molecule to the effector cell results in a targeted effector cell i.e., an effector cell with attached bispecific antibody or heteroantibody containing antigen binding regions which are specific for a desired pathogen. The targeted effector cells can be used to bring about antibody dependent cell mediated cytolysis (ADCC) and/or phagocytosis of the target cells in vivo.

The bispecific molecules of this invention have at least two distinct binding specificities. The molecules contain a binding specificity for a pathogen such as a virus component, and a binding specificity for the Fcγ receptor of effector cells.

The Fc-receptor binding specificity is provided by a binding agent which binds to the high affinity (p72) Fcγ receptor (FcRI) for human IgG without being blocked by human IgG. The preferred Fcγ receptor binding agent is an antibody, antibody fragment, antibody variable region, or genetic construct having the following characteristics:
a. it reacts specifically with the high affinity Fcγ receptor;
b. it reacts with the receptor through its antigen combining region independent of any Fc portion;
c. it reacts with an epitope of Fcγ receptor which is distinct from the Fc binding (i.e. ligand binding) site of the receptor; and
d. it binds ligand-occupied receptor.

The anti-Fcγ receptor antibodies of this invention can be produced as described in U.S. Patent Application Serial Number 151,450; Fanger et al., "Monoclonal Antibodies to Fc Receptors for Immunoglobulin G on Human Mononuclear Phagocytes", the teachings of which are incorporated by reference herein.

The binding specificity for the pathogen component can be any binding agent specific for an antigen of the pathogen. For example, if the targeted pathogen is a virus, viral antigens such as those associated with Epstein Barr virus (EBV glycoprotein: M. Mackett and J.R. Arrand, EMBO J., 4: 3229-3234 (1985)); human Influenza virus (Haemagglutinin: E.B. Stephens et al., EMBO J., 5: 237-245 (1986)); hepatitis B virus (HBV major surface antigen: R.H. Purcell and J.L. Gerin, Am. J. ed. Sci., 270: 395-399 (1975)); and HIV (capsid env glycoproteins: A.S. Fauci, Science, 239: 617-622 (1988)) can be used as the source of viral target antigen needed to produce the binding specificity for molecules of this invention.

In preferred embodiments for HIV treatment, the HIV component is the envelope glycoprotein gp120 of HIV, found in the viral envelope and in cells harboring infectious HIV. The bispecific molecules are specific for gp120 and the HIV-binding agent can be provided by naturally-ocurring or recombinant forms of the CD4 receptor of T cells or by the HIV binding domain of CD4. It is well known that CD4, expressed on T-lymphocytes, is the receptor for the HIV envelope glycoprotein gp120. The CD4 protein is also the primary receptor for HIV entry into host cells, and for membrane fusion which contributes to cell-to-cell transmission of HIV and to its cytopathic effects. Maddon, P.J. et al ., Cell, 47: 333-348 (1986). Since the CD4 antigen was identified as the cell-surface receptor for HIV, it has been repeatedly shown that soluble forms of CD4 antigen can block the infectivity of the virus. Traunecker, A. et al., Nature, 331: 84-86 (1988). Soluble CD4 inhibits diverse variants of HIV, indicating that all these viruses may share a relatively conserved CD4-binding region.

Soluble CD4 analogs or CD4 fragment with an affinity for gp120 comparable to that of intact CD4 can be prepared using methods described in the art. See, for example, Berger, E.A. et al., Proc. Nat'l. Acad. Sci. USA, 85: 2357-2361 (1988); Arthos, J., et al., Cell, 57: 469-481 (1989). Soluble CD4 fragments lack the hydrophobic transmembrane portion or contain only a small fraction of this transmembrane portion. Soluble CD4 fragments and CD4 analogs can be produced by inserting truncated CD4-encoding cDNA into expression vectors. CD4 polypeptide can be produced by such cells and the soluble CD4 can be tested for its ability to bind gp120 using standard coimmunoprecipitation assays. See, for example Smith, D.H. et al., Science 238, 1704-1707 (1987).

Alternatively, the HIV binding specificity of the molecules of this invention can be provided by anti-gp120 antibodies. These antibodies can also be produced by conventional monoclonal antibody methodology, e.g. the standard somatic cell hybridization technique of Kohler and Milstein, Nature, 256, 495 (1975), using the gp120 glycoprotein, or fragments thereof, as the immunogen. In brief, an animal such as a mouse is immunized with gp120 of HIV. The gp120 can be purified, or partially purified from viral lysates for this purpose. The purification of gp120 can be accomplished by affinity chromatography with antibody against gp120. After immunization, B cells are taken from the immunized animal and then fused with an immortalizing cell such as a myeloma cell. See, for example, M.S.C. Fung et al., Biotechnology, 5: 940-946 (1987). It will be appreciated that subunits of gp120 can also be employed as the HIV component to which a binding specificity is provided. For example, antibodies can be prepared against the gp41 transmembrane protein as well as smaller gene products of the envelope gene of HIV. See, for example, W.G. Robey et al., Science 228, 593-595 (1985).

Bispecific molecules of this invention can also be prepared by conjugating a binding specificity for a pathogen (i.e. virus or viral antigen) to an anti-Fcγ receptor (FcγR) gene. Development and cloning of the gene for the binding site of anti-FcγR, is well within the capabilities of those skilled in the art. This gene could be linked to genes encoding viral receptors such as the CD4 molecule. Such constructs can be used to target viral infectious agents and infected cells through FcγR.

The bispecific molecules of this invention can be of several configurations. Bispecific antibodies are single antibodies (or antibody fragments) which have two different antigen binding sites (variable regions). Bispecific antibodies of this invention have one binding site for Fcγ receptor and one binding site for a viral epitope. Bispecific antibodies can be produced by chemical techniques (see e.g., Kranz, D. M. et al., Proc. Natl. Acad. Sci. USA 78,5807 (1981)) by "polydoma" techniques (see U.S. Patent 4,474,893, to Reading) or by recombinant DNA techniques.

Heteroantibodies are two or more antibodies, or antibody binding fragments (Fab) linked together, each antibody or fragment having a different specificity. Bivalent heteroantibodies of this invention comprise an antibody (or fragment) specific for Fcγ receptor, coupled to an antibody (or fragment) specific for a viral epitope. Heteroantibodies can be prepared by conjugating Fcγ receptor antibody with antibody specific for an epitope of the HIV envelope glycoprotein gp120. A variety of coupling or crosslinking agents can be used to conjugate the antibodies. Examples are protein A, carboiimide, dimaleimide, dithio-bis-nitrobenzoic acid (DTNB), and N-succinimidyl-3-(2-pyridyldithio) propionate (SPDP). SPDP and DTNB are the preferred agents; procedures for crosslinking antibodies with these agents are known in the art. See e.g., Karpovsky, B. et al., (1984) J. Exp. Med. 160:1686; Liu, M.A. et al., (1985) Proc. Natl. Acad. Sci USA 82:8648; Segal, D.M. and Perez, P., U.S. Patent No. 4,676,980 (June 30, 1987); and Brennan, M. Biotechniques 4:424 (1986).

The bispecific molecules of this invention can also be prepared as recombinant molecules. Constructs can be developed that comprise genes encoding viral receptors linked to genes encoding the binding site (variable region) of anti-FcγR antibody. Thus, a recombinant nucleic acid which encodes a molecule having dual specificity can be prepared by linking a gene encoding a receptor for a viral antigen (e.g. a cell-surface receptor such as CD4 which binds to gp120 on HIV or HIV-infected cells) to the gene encoding either the light or heavy chain variable region of an anti-FcγR antibody. These genetic constructs, or other constructs linking genes for different viral receptors to the anti-FcγR antibody gene, can be expressed in suitable host cells.

Bispecific molecules of this invention can be administered to target the killing of virus and virally infected cells. The molecules can be given in free form. Alternatively, the molecules can be attached to the surface of effector cells in vitro and the cells can be administered. In each mode the principle is the same; the effector cell is targeted toward the virus.

Effector cells for targeting are human leukocytes, preferably macrophages. Other cells can include monocytes, activated neutrophils, and possibly activated natural killer (NK) cells and eosinophils. Macrophages can be treated with IFN-γ before targeting to increase the number of Fc receptors for attachment of the targeting antibody or heteroantibody. Neutrophils and NK cells can also be activated with IFN-γ in this way. The effector cells may also be activated before targeting by other cytokines such as tumor necrosis factor, lymphotoxin, colony stimulating factor, and interleukin-2. If desired, effector cells for targeting can be obtained from the host to be treated, or any other immunologically-compatible donor.

The targeted effector cells can be administered as a suspension of cells in a physiologically acceptable solution. The number of cells administered can be in the order of 10⁸-10⁹, but will vary depending on the therapeutic purpose. In general, the amount will be sufficient to obtain localization of the effector cell at the target cell or pathogen, and to effect killing of the cell or pathogen by antibody dependent cell-mediated cytolysis (ADCC) and/or phagocytosis. Routes of administration can also vary. The targeted effector cells could be administered intravenously, intramuscularly, or intraperitoneally.

Bispecific molecules of this invention link viral-specific binding agents to FcγRI on effector cells in such a way that the large excess of human IgG in vivo does not interfere with binding of the molecule to effector cells or interfere with functioning of effector cells. This is possible because the anti-FcγR component of these molecules binds to FcγR at an epitope outside of its ligand binding domain. Effector cells (i.e. macrophages) targeted in this way can be employed to bring about antibody-dependent cell-mediated killing of HIV or HIV-infected cells.

The bispecific molecules of this invention have a potentially long half-life in vivo. This can result from the interaction of these constructs with FcγR on all monocytes and macrophages where it might remain for long periods of time, much of it out of circulation, but functionally active throughout the body on all cells of the reticuloendothelial system.

Bivalent bispecific molecules of this invention can be more sensitive to triggering than other constructs because of their bivalent nature. This is because internalization of the construct and killing of the targeted infectious agent requires receptor crosslinking. A bivalent bispecific complex will initiate cross-linking more efficiently that a monovalent bispecific construct. Furthermore, the binding avidity of a bivalent bispecific construct is likely to be greater than a monovalent bispecific molecule, and therefore be more effective in clearing HIV and HIV-infected cells. This is an important advantage of a bivalent bispecific molecule. A monovalent molecule comprising, for example, the Fc region of IgG complexed with a viral binding specificity (Capon, D.J. et al., supra) will bind to only one FcγRI molecule since only one of the Fc regions of an antibody can bind to the high-affinity FcγRI receptor. Constructs of this invention having bivalent bispecific or heteroantibody configurations offer an advantage since they can be manipulated to provide greater avidity or triggering capability.

The bispecific molecules of this invention are specific for interaction with only FcγRI. Constructs employing the Fc domain of IgG (Capon, D.J. et al., supra) interact with all three types of Fc receptor. This lack of specificity may be of considerable disadvantage since FcγRII and FcγRIII are expressed by other cells besides monocytes, such as B-cells, platelets, and placental Ig transfer cells. Thus, there is the possibility that HIV may be introduced through FcγRII and/or FcγRIII into cells that cannot kill but which may harbor the virus. Moreover, FcγRI has been found to be a killing receptor on all cell populations on which it has been found. In contrast, the other two Fc receptors only function as cytotoxic trigger molecules on some of the cells on which they are expressed, and then only under some conditions.

Those skilled in the art will recognize, or be able to ascertain using no more than routine experimentation, many equivalents to the specific embodiments of the invention described herein. Such equivalents are intended to be encompassed by the following claims.

In the invention the following embodiments are disclosed:

(1) the bispecific molecule of claim 2, wherein the virus is human immunodeficiency virus (HIV) and optionally wherein
   a) the virus component is the envelope glyco-protein gp120 of HIV or a fragment thereof; or
   b) the viral component is the envelope glycoprotein gp41 of HIV; and further optionally the binding specificity for the viral component gp120 is provided by the CD4 receptor of T-cells or the gp120 binding domain thereof; or the binding specificity for the viral component gp120 is provided by a gp120-specific antibody or fragment thereof.
(2) A bispecific molecule comprising a specific binding agent for human immunodeficiency virus (HIV) and a specific binding agent for the high affinity Fcγ receptor for IgG on human monocytes, the binding site for the agent on the high-affinity Fcγ receptor being distinct from the ligand binding site of the receptor for Fc; and optionally wherein
   a) the specific binding agent for HIV binds to the envelope glycoprotein gp120 or a fragment thereof; or
   b) the specific binding agent for HIV binds to the envelope glycoprotein gp41 of HIV; or
   c) the specific binding agent is the CD4 receptor of T-cells; or
   d) the specific binding agent is anti-gp120 antibody.
(3) A heteroantibody, comprising:
   a) an antibody or antibody binding fragment specific for the envelope gp120 glycoprotein of the HIV virus; and
   b) an antibody or antibody binding fragment specific for the high-affinity Fcγ receptor for IgG on human effector cells, the binding of which to the human Fc receptor of the effector cells is not blocked by human immunoglobulin G; and optionally wherein the effector cell is selected from the group consisting of monocytes, macrophages, neutrophils and eosinphils.

## Claims

1. A bispecific molecule having a binding specificity for a pathogen or pathogen component and a binding specificity for the high-affinity Fcγ receptor, the binding of which to the Fcγ receptor is not blocked by human immunoglobulin G.

2. A bispecific molecule of claim 1, wherein the pathogen or pathogen component is a virus or viral component.

3. A bispecific molecule of claim 1, which is
a) a bispecific antibody; or
b) an aggregate of two or more antibodies or fragments thereof; or
c) a recombinant molecule.

4. A bispecific reagent, comprising a CD4 receptor linked to an antibody or fragment thereof specific for an epitope of the high affinity Fcγ receptor, the epitope being outside of the ligand binding domain for Fc of the receptor and the binding of which to the Fc receptor is not blocked by human immunoglobulin G.

5. A target-specific effector cell, comprising:
a) an effector cell expressing high affinity receptor for the Fc portion of IgG; and
b) a bispecific molecule bound to an epitope of the Fc receptor of the effector cell that is outside of the ligand binding domain of the receptor, the molecule comprising:
i) at least one binding specificity for a virus or viral component; and
ii) at least one binding specificity for the high-affinity Fcγ receptor, the binding of which to the Fc receptor of the effector cell is not blocked by human immunoglobulin G.

6. A target-specific effector cell of claim 5, wherein
a) the effector cell is a human monocyte or macrophage; or
b) the virus is human immunodeficiency virus (HIV).

7. A target-specific effector cell of claim 5, wherein the viral component is either (i) the envelope glycoprotein gp120 of HIV, and optionally (a) the binding specificity for a viral component is provided by the CD4 region of T-cells or the gp120 binding domain thereof, or (b) the binding specificity for a virus component is provided by a gp120-specific antibody or fragment thereof; or (ii) the viral component is the envelope glycoprotein gp41 of HIV.

8. A target-specific effector cell of claim 5, wherein
a) the bispecific molecule is a bispecific antibody; or
b) the bispecific molecule is an aggregate of two antibodies or fragments thereof.

9. A bispecific molecule according to any one of claims 1 to 4 for use in the treatment of viral infection.

10. A target-specific effector cell according to any one of claims 5 to 8 for use in the treatment of viral infection.
